# EUROPÄISCHE PATENTSCHRIFT

(11) **EP 0 583 691 B1**
(45) Veröffentlichungstag und Bekanntmachung des Hinweises auf die Patenterteilung: **19.03.1997**
(21) Anmeldenummer: 93112538.9
(22) Anmeldetag: 05.08.1993
(51) Int. Cl.: B04B 5/04

(54) **Verfahren und Vorrichtung zur kontinuierlichen Aufbereitung einer Zellsuspension**
Method and device for a continuous treatment of a cellular suspension
Procédé et dispositif pour le traitement continu d'une suspension cellulaire

(30) Priorität: 14.08.1992 DE 4226974
(43) Veröffentlichungstag der Anmeldung: 23.02.1994
(73) Patentinhaber: Fresenius AG, D-61350 Bad Homburg (DE)
(72) Erfinder: Biesel, Wolfgang, Dr., D-55606 Ottweiler (DE); Mathieu, Bernd, Dr., D-66583 Spiesen-Elversberg (DE); Weber, Wolfram, D-66583 Spiesen-Elversberg (DE)
(74) Vertreter: Fuchs, Luderschmidt & Partner Patentanwälte

(56) Entgegenhaltungen:
- EP-A- 0 026 334
- EP-B- 0 155 684
- DE-C- 3 817 664
- US-A- 3 825 175

## Beschreibung

Die Erfindung bezieht sich aus ein Verfahren zur kontinuierlichen Aufbereitung einer Zellsuspension, bei welchem die Zellsuspension zentrifugiert wird und die abgetrennten Bestandteile der Zellsuspension separat abgeführt werden und bei dem kontinuierlich bestimmte Zellen der Zellsuspension konzentriert werden.

Die Erfindung bezieht sich weiterhin auf eine Vorrichtung zur Durchführung eines Verfahrens zur Aufbereitung einer Zellsuspension, insbesondere des oben genannten Verfahrens, mit einer Zentrifuge, welche zumindestens eine Separationskammer, eine Zulaufleitung für die Zellsuspension, eine Ablaufleitung für ein zu gewinnendes, hochreines Zellkonzentrat, zumindest eine Ablaufleitung für nicht benötigte Bestandteile und zumindest eine Zulaufleitung für eine Waschlösung aufweist.

Aus dem Stand der Technik sind vielfältige derartige Separationseinrichtungen und zugehörige Verfahren bekannt, bei welchen insbesondere Blut in seine Bestandteile getrennt und diese, beispielsweise Erythrozyten oder Plasma, einer weiteren Verwendung zugeführt werden.

Es gibt vielfältige medizinische Anwendungsfälle für derartige Verfahren und Vorrichtungen. Eines dieser Anwendungsgebiete ist die intraoperative Autotransfusion, welche eine fremdblutsparende Transfusionstechnik darstellt, welche in den letzten 10 Jahren breite Anwendung gefunden hat. Die intraoperative Autotransfusion ist ein Verfahren, das die Retransfusion von aus dem Operationsfeld gesammeltem Blut ermöglicht. Anwendung im Bereich der intraoperativen Autotransfusion finden sogenannte "Vollblutretransfusionsverfahren", die das gesammelte Blut lediglich einer Partikelfiltration unterziehen, bis hin zu Plasmaseparations-/Waschverfahren, die ein gewaschenes Erythrozytenkonzentrat zur Reinfusion bereitstellen. Die Vorteile der Transfusion von autologen, d.h. patienteneigenem Blut liegen, gegenüber der Transfusion von homologem (Fremd-) Blut in der Vermeidung von Infektionskrankheiten, wie beispielsweise AIDS, Hepatitis oder andere, sowie in der Vermeidung von Transfusionsreaktionen aufgrund biologischer Inkompatibilität und Immunsystemreaktionen.

Im Rahmen der Entwicklung intraoperativer Autotransfusionstechniken hat es sich herausgestellt, daß die Transfusion von Vollblut gegenüber der Transfusion gewaschener Erythrozytenkonzentrate nachteilig sein kann. Diese Nachteile der Vollbluttransfusionsverfahren bestehen darin, daß unerwünschte Bestandteile des abgesammelten Blutes nicht eliminiert werden können. Intraoperatives Blut enthält unbekannte Mengen an Hämolyseprodukten, aus dem Gewebe eingeschwemmten oder von außen zugeführten Fremdbestandteilen, überschüssiges Volumen, Antikoagulantien, aktivierte plasmatische und zelluläre Gerinnungsfaktoren, Produkte der Gewinnungsaktivierung und des fibrinolytischen Systems. All diese Bestandteile können klinische Komplikationen auslösen, was zu Einschränkung des Einsatzgebietes geführt hat. Es ist aus dem Stand der Technik bekannt, bei derartigen Autotransfusionssystemen von Blut Filtersysteme einzusetzen, welche jedoch lediglich Blutgerinsel oder Gewebeteile zurückhalten. Ein derartiges System ist aus der US-PS 4,014,329 bekannt. Die US-PS 4,886,487 beschreibt eine Vorrichtung zur Absonderung überschüssigen Fluids, wobei jedoch Gerinnungsfaktoren, Waschflüssigkeit, Antikoagulans und andere Additive mit dem Blut dem Patienten rückgeführt werden.

Als Alternative zur Vollblutretransfusion wurden Plasmaseparations- (kombiniert mit) / Waschverfahren entwickelt, welche Zentrifugen verwenden. Derartige Zentrifugen sind in der DE-OS 22 62 856 und der WO 89/01792 beschrieben. Diese Zentrifugen weisen jedoch den Nachteil auf, daß sie diskontinuierlich arbeiten. Die einzelnen Verfahrensschritte der Blutaufbereitung und Retransfusion laufen zeitlich aufeinanderfolgend ab, wobei die Aufbereitung in jeweils relativ großen Einheiten mit vollständig gefüllter Kammer erfolgen muß, um die Funktionsfähigkeit des Verfahrens sicherzustellen. So ist es beispielsweise erforderlich, 225 ml Erythrozytenkonzentrat in einer Zentrifuge anzusammeln.

Die Nachteile derartiger diskontinuierlicher Verfahrensweisen liegen vor allem in dem großen Startvolumen, welches für die jeweilige Aufbereitung erforderlich ist. Die Aufbereitung kleiner Mengen, beispielsweise in der Pädiatrie scheidet somit gänzlich aus. Ein weiterer, wesentlicher Nachteil derartiger diskontinuierlicher Verfahrensweisen ergibt sich aus der langsamen Prozessgeschwindigkeit, welche durch die zeitliche Aufeinanderfolge der einzelnen Verfahrensschritte bedingt ist.

Es ist somit festzustellen, daß die bekannten Verfahren und Vorrichtungen zu einer kontinuierlichen Aufbereitung einer Zellsuspension, wie sie bei einer intraoperativen Autotransfusion erforderlich ist, nicht geeignet sind.

Die DE-PS 38 17 664, die die Merkmale des Oberbegriffs der Ansprüche 1 und 8 zeigt, beschreibt eine Gegenstromextraktionszentrifuge, bei welcher Vollblut im Gegenstrom gegen eine Waschlösung geschleudert wird. Weder die Vorrichtung noch das Verfahren erfüllen jedoch die Anforderungen, welche im Rahmen einer Autotransfusion gestellt werden, da die unerwünschten Bestandteile nicht in sicherer und effektiver Weise abgetrennt werden können.

Die EP-B1-155 684, beschreibt eine Vorrichtung zur Trennung von Blut, welche kontinuierlich arbeitet und eine Rückführung von thrombozytenarmem Plasma vorsieht, um das zugeführte Vollblut zu verdünnen. Für die geforderten Anwendungsgebiete ist diese Vorrichtung jedoch nicht einsetzbar, da hier in der Separationskammer kein entsprechendes Zuleitungssystem vorgesehen ist.

Die US-PS 4,010,894 zeigt eine Zentrifuge mit einem Ringkanal, welcher eine mehrstufige Arbeitsweise zuläßt und die getrennte Abfuhr von roten Blutzellen, Plasma und weiteren Blutbestandteilen ermöglicht. Auch diese Vorrichtung ist für die erfindungsgemäße Anwendung nicht anwendbar, da keine ausreichende Abtrennung nicht gewünschter Bestandteile der Zellsuspension gewährleistet ist. Das Zellkonzentrat kann keinem Waschvorgang unterzogen werden, was zur ausreichenden Abtrennung unumgänglich ist.

Der Erfindung liegt die Aufgabe zugrunde, ein Verfahren und eine Vorrichtung der eingangs genannten Art zu schaffen, welche bei einfachem Aufbau und einfacher, betriebssicherer Anwendbarkeit eine wirkungsvolle Abtrennung unerwünschter Bestandteile der Zellsuspension ermöglichen, welche kontinuierlich arbeiten und bei welchen auch die Behandlung kleiner Volumina von Zellsuspensionen möglich ist, wobei die Aufbereitung mit hoher Effizienz und in kurzer Zeit durchgeführt wird.

Erfindungsgemäß wird die Aufgabe hinsichtlich des Verfahrens dadurch gelöst, daß die konzentrierten Zellen in einer Waschlösung resuspendiert werden und daß Restbestandteile von den Zellen abgetrennt werden.

Das erfindungsgemäße Verfahren zeichnet sich durch eine Reihe erheblicher Vorteile aus. Erfindungsgemäß ist somit ein kontinuierliches Verfahren zur Abtrennung einer Zellsorte geschaffen, bei welchem die gewünschte Zellsorte, vorzugsweise Erythrozyten mittels Zentrifugation von der Zellsuspension getrennt werden. Nachfolgend wird das Zellkonzentrat durch Resuspension in der Waschlösung gewaschen. Nachfolgend wird die verunreinigte Waschlösung durch weitere Zentrifugation abgetrennt. Es ist somit auf sehr wirkungsvolle Weise möglich, die gewünschten Bestandteile der Zellsuspension in einer hohen Konzentration und in einem hohen Reinheitsgrad zu erhalten. Somit ist das Verfahren insbesondere zur intraoperativen Aufbereitung von Blut für die gefahrlose Reinfusion geeignet. Das erfindungsgemäße Verfahren arbeitet kontinuierlich und benötigt nur kleine Startvolumina, es ist möglich, eine hohe Blutdurchsatzrate und somit eine hohe Produktionsrate von Erythrozytenkonzentrat (60-100 ml/min) bei niedrigen, blutschonenden Förderraten zu realisieren. Weiterhin ist es möglich, das Erythrozytenkonzentrat zur direkten Reinfusion zu verwenden. Ein weiterer, wesentlicher Vorteil des dreistufigen, kontinuierlich arbeitenden Verfahrens besteht in der Verkürzung des Zeitablaufes des Gesamtprozesses um mindestens den Faktor 2.

Gemäß des erfindungsgemäßen Verfahrens ist vorgesehen, daß die Konzentrierung der Zellen auf Hämokritwerte von 50 bis 70% erfolgt. Hierdurch wird eine Abtrennung von mindestens 95% der ursprünglichen Plasmamenge des zugeführten Vollblutes erreicht.

Als waschlösung kann isotonische Kochsalzlösung, Ringerlösung oder ähnliches verwendet werden.

Das erfindungsgemäße Verfahren wird üblicherweise wiefolgt durchgeführt:

Blut, typischerweise mit einem Hämokrit von 10 bis 30%, wird aus dem Operationsfeld angesaugt, antikoaguliert, filtriert und in einem Behältnis zwischengelagert. Das Blut wird über eine Zufürungsleitung in eine Separationskammer gegeben und entsprechend der Dichte in seine Bestandteile zerlegt und absepariertes Plasma abgetrennt. In einem zweiten Verfahrensschritt wird die konzentrierte Zellfraktion (vornehmlich Erythrozytenkonzentrat) durch kontinuierliche Zugabe einer Waschlösung resuspendiert. Durch die nachfolgende nochmalige Separation werden die verbliebenen nicht-zellulären Bestandteile abgetrennt und durch die Abführung der verunreinigten Waschlösung verbleibt als Ergebnis sehr reines Erythrozytenkonzentrat.

Erfindungsgemäß wird die Aufgabe hinsichtlich der Separationsvorrichtung dadurch gelöst, daß die Separatiorskammer im wesentlichen in Form eines Ringkanals mit im wesentlichen rechteckförmigen Querschnitt ausgebildet ist, wobei die radiale Abmessung des Ringkanals (Breite) kleiner ist als der mittlere Durchmesser des Ringkanals, daß der Ringkanal in Umtangrichtung an zwei Enden geschlossen ist, wobei an einem Ende die Zulaufleitung für die Zellsuspension und am anderen Ende die Ablaufleitung für das zu gewinnende, hochreine Zellkonzentrat angeschlossen sind, und daß der Ringkanal in Umfangsrichtung in drei Bereiche unterteilt ist, von denen ein erster Bereich einer ersten Separation der Zellsuspension, ein zweiter Bereich der Resuspendierung in einer Waschlösung und ein dritter Bereich einer zweiten Separation der resuspendierten Zellen dient.

Dabei ist bevorzugt vorgesehen, daß die Zulaufleitung für die Waschlösung zur Durchleitung der Waschlösung im Gegenstromverfahren stromauf oder stromab der Ablaufleitung die verunreinigte Waschlösung angeordnet ist.

Unter dem Gesichtspunkt einer platz- und materialsparenden Konstruktion ist es dabei besonders vorteilhaft, wenn die Ablaufleitung für die verunreinigte Waschlösung einstückig mit der Ablaufleitung für die nicht benötigten Bestandteile ausgebildet ist.

Von den möglichen geometrischen Ausgestaltungen haben sich diejenigen als besonders vorteilhaft herausgestellt, bei denen die Separatiohkammer radiärsymmetrisch oder spiralisch oder doppelspiralisch ausgebildet ist. Die Zulaufleitung für die Waschlösung kam so angeordnet sein, daß sich eine Strömung in radialer Richtung von außen nach innen, entgegen dem Schwerefeld, ergibt.

Die Separationskammer kann vorteilhafterweise entweder einteilig oder zweiteilig ausgebildet sein, wobei bei der zweiteiligen Ausbildung der Separationskammer die beiden Teile vorteilhafterweise durch eine Leitung miteinander verbunden sind und die Zulaufleitung für die Waschlösung im Bereich dieser Leitung mündet.

Die Separationskammer selbst ist vorteilhafterweise selbst tragend und starr ausgebildet, so daß weitere Lagerbauteile etc. entfallen können. Für den Krankenhausbetrieb hat es sich aus Hygiene- und Kostengründen als vorteilhaft herausgestellt, wenn die Separationskammer als wiederverwertbares Einweg-Teil ausgebildet ist. Auf diese Weise können die Werkstoffe der Separationskammer wiederverwertet werden und trotzdem fallen für die Separationskammer selbst keine unhygienischen Reinigungsarbeiten etc. an.

Eine besonders strömungsgünstige Ausgestaltung der Zulaufleitung besteht darin, daß die Zulaufleitung für die Waschlösungen im Anschlußbereich in den Ringkanal der Separationskammer kegelförmig sich erweiternd ausgebildet ist. Dabei erstreckt sich der kegelförmig sich erweiternde Bereich der Zulaufleitung vorteilhafterweise über die gesamte Höhe des Ringkanals und ist mittels eines Hohlstegs mit diesem verbunden. Die Strömungsform ist dabei besonders günstig, wenn im Bereich der Mündung des den kegelförmigen Bereich der Zulaufleitung mit dem Ringkanal verbindenden Hohlstegs in Richtung des Separationsverlaufs hinter der Mündung eine in den Innenraum des Ringkanals gerichtete Einwölbung angeordnet ist.

Im folgenden wird die Erfindung bezüglich der Separationsvorrichtung an Hand von Ausführungsbeispielen in Verbindung mit der Zeichnung beschrieben. Dabei zeigt:
- Fig. 1: eine schematische Draufsicht auf ein Ausführungsbeispiel der erfindungsgemäßen Separationskammer,
- Fig. 2: eine schematische abgewickelte Funktionsdarstellung der Abläufe in der in Fig. 1 gezeigten Separationskammer.
- Fig. 3: eine abgewickelte, schematische Darstellung der Funktionsabläufe eines weiteren Ausführungsbeispieles einer erfindungsgemäßen Separationskammer,
- Fig. 4: eine Draufsicht auf ein konstruktives Ausführungsbeispiel der erfindungsgemäßen Separationskammer,
- Fig. 5: eine vergrößerte Darstellung der Ausgestaltung des Einlaufs für die Waschlösung in teil-perspektivischer Darstellung, und
- Fig. 6: eine Seitenansicht der in Figur 5 gezeigten Ausgestaltung.

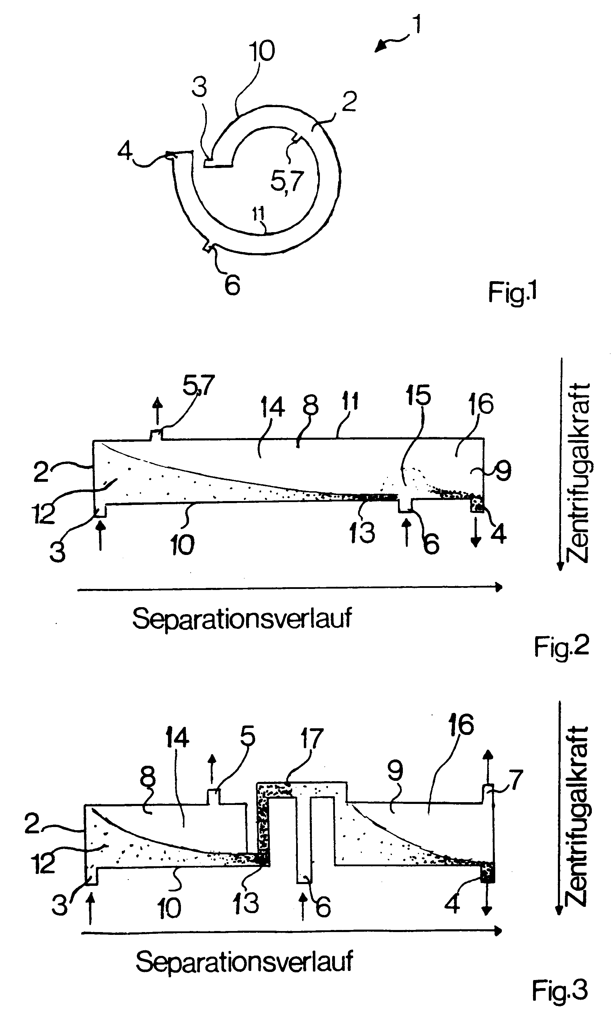

In Fig. 1 ist in schematischer Weise eine Separationskammer 1 dargestellt, welche einen spiralförmigen Ringkanal 2 umfaßt. Dieser Ringkanal kann radiärsymmetrisch oder spiralförmig ausgebildet sein. Z. B. an einer Außenseite 10 des Ringkanals 2 ist hier eine Zulaufleitung 3 für Zellsuspension, beispielsweise Vollblut, angeordnet. An der Außenseite 10 des Ringkanales 2 ist eine Ablaufleitung 4 für Zellkonzentrat (Erythrozytenkonzentrat) angeordnet, wobei die Abführung der Erythrozyten in Richtung der Zentrifugalkraft erfolgt. Im mittleren Bereich bzw. im Endbereich des Ringkanales 2 ist eine Zulaufleitung 6 für Waschlösung vorgesehen, wobei die Waschlösung entgegen der Zentrifugalkraft zugeführt wird. An einer Innenseite 11 des Ringkanales 2 ist eine Ablaufleitung 5 für nicht benötigte Bestandteile (Plasma) vorgesehen, welche einstückig mit einer Ablaufleitung 7 für verunreinigte Waschlösung ausgebildet ist. Diese Auslässe können in der Mitte des Ringkanales 2 liegen, sie können jedoch auch in dem Bereich bis zum Auslaß des gereinigten Erythrozytenkonzentrates und bis zum Bluteinlaß angeordnet werden.

Die Fig. 2 zeigt den Trennungsverlauf des Blutes der in Fig. 1 gezeigten Separationskammer, wobei in der abgewickelten Darstellung der Verlauf der Separation gegen die Zentrifugalkraft aufgetragen ist. Im linken Bildbereich der Fig. 2 ist die Zufuhr von Vollblut durch die Zulaufleitung 3 dargestellt, das Vollblut 12 sammelt sich in dem Ringkanal 2 und wird durch den Einfluß der Zentrifugalkraft in einem ersten Bereich 8 aufgetrennt, die Erythrozyten 13 setzen sich an der Außenseite 10 des Ringkanales 2 ab. Demgegenüber wird Plasma 14 kontinuierlich durch die Ablaufleitung 5 abgeführt.

In dem Bereich, in welchem das Erythrozytenkonzentrat einen Hämokrit von mehr als 60% aufweist, wird, ebenfalls entgegen der Zentrifugalkraft, durch die Zulaufleitung 6 Waschlösung zugeführt, welche durch die radiale Zuführung in einem Mischbereich 15 in optimaler Weise mit dem Erythrozytenkonzentrat gemischt wird.

Im weiteren Verlauf der Trennung werden die Erythrozyten 13 in einem zweiten Bereich 9 von der verunreinigten Waschlösung 16 getrennt, welche durch die Ablaufleitung 7 abgeführt wird. Die Ablaufleitungen 5 und 7 sind hier einstückig ausgebildet. Das gereinigte Erythrozytenkonzentrat wird durch die Ablaufleitung 4 abgezogen und dem Patienten zugeführt.

Die Fig. 3 zeigt ein abgewandeltes Ausführungsbeispiel, bei welchem die Kammer zweistufig ausgebildet ist. Wie bei dem ersten Ausführungsbeispiel der Fig. 2 wird durch die Zulaufleitung 3 Vollblut (12) von der Außenseite 10 der Ringkammer 2 zugeführt. Das Erythrozytenkonzentrat 13 wird über eine Leitung 17 abgezogen, während das Plasma 14 über die Ablaufleitung 5 abgeführt wird. Im Bereich der Leitung 17 ist die Zulaufleitung 6 für Waschlösung vorgesehen,in der diese mit EK vermischt wird. Nach der nachfolgenden Trennung im 2. Teilbereich 9 wird das Erythrozytenkonzentrat durch die Ablaufleitung 4 abgeführt, während die verunreinigte Waschlösung 16 durch die Ablaufleitung 7 abgeführt wird.

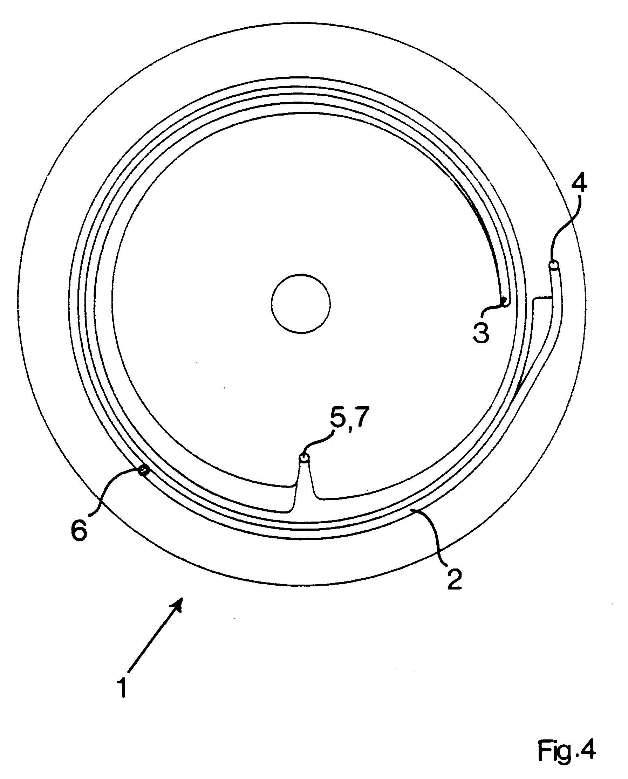

Die Fig. 4 zeigt in der Draufsicht ein konstruktives Ausführungsbeispiel einer spiralförmig ausgebildeten Separationskammer 1, wobei dieses Ausführungsbeispiel im wesentlichen dem Aufbau des in den Fig. 1 und 2 gezeigten Ausführungsbeispieles entspricht.

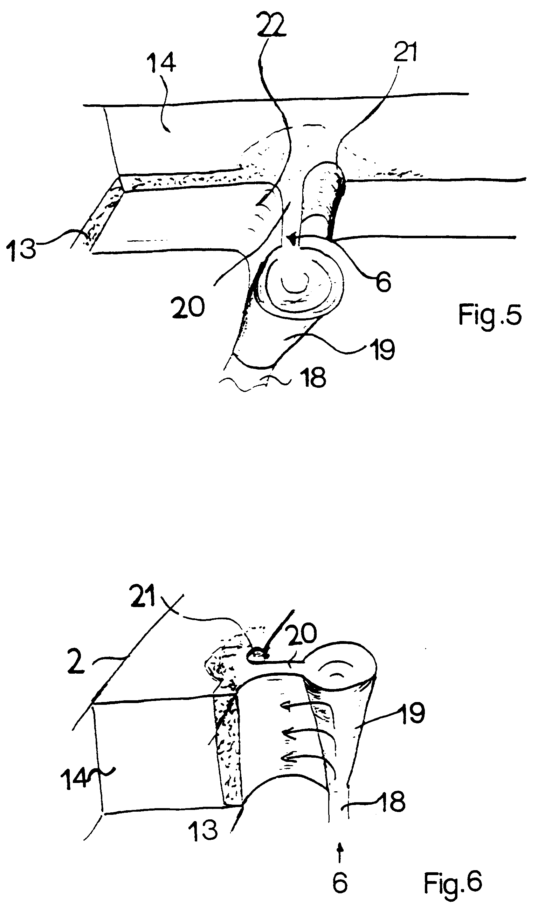

Die Figuren 5 und 6 zeigen in perspektivischer Teilansicht die Ausgestaltung der Zulaufleitung 6 für die Waschlösung. Zur Verdeutlichung sind die Erythrozyten 13 sowie das Plasma 14 abgebildet, wobei zur Verdeutlichung insbesondere auf die Darstellung der Figur 2 zu verweisen ist. Bei der in den Figuren 5 und 6 gezeigten Ausgestaltungsform wird Waschlösung durch einen Schlauch oder ein Rohr 18 einem kegelförmigen Körper 19 zugeführt, wobei die Zuleitung in den unteren, einen reduzierten Durchmesser aufweisenden Bereich des kegelförmigen Körpers 19 erfolgt. Dieser ist über einen plattenartigen Steg, welcher selbstverständlich hohl ausgebildet ist, mit dem Innenraum der Separationskammer verbunden. In Richtung des Separationsverlaufs (siehe Figur 2) schließt sich an den hohlen Steg 20 eine in den Innenraum des Ringkanals 2 gerichtete Einwölbung 21 an, welche in gewissem Rahmen eine Barriere bildet. Demgegenüber ist der der Barriere 22 gegenüberliegende Rand des hohlen Steges abgerundet. Diese Ausgestaltungsform ermöglicht ein gleichmäßiges Einströmen der Waschlösung über die Höhe des gesamten Spaltes. Dies führt zu einer turbulenten Strömung und damit zu einer gleichmäßigen Durchmischung. Der hohle Steg oder Spalt 20 ist beispielsweise 0,5 bis 2,0 mm breit.

Die Erfindung ist nicht auf die gezeigten Ausführungsbeispiele beschränkt, vielmehr ergeben sich im Rahmen der Erfindung vielfältige Abwandlungs- und Modifikationsmöglichkeiten. Diese sind insbesondere hinsichtlich der Effektivität des Waschprozesses möglich, um einen innigen Kontakt zwischen den Zellen und der Waschlösung, d.h. eine möglichst vollständige Vermischung zu gewährleisten. Bezüglich der Menge der Waschlösung ergeben sich unterschiedlichste Dosierungsmöglichkeiten in Abhängigkeit von dem jeweiligen Anwendungsfall, im Normalfall wird etwa die zehnfache Menge des verbleibenden Restplasmas an Waschlösung eingesetzt. Bei stark verunreinigtem oder geschädigtem Blut sind jedoch auch andere Verdünnungsfaktoren vorteilhaft.

## Patentansprüche

1. Verfahren zur kontinuierlichen Aufbereitung einer Zellsuspension, bei welchem die Zellsuspension zentrifugiert wird und die abgetrennten Bestandteile der Zellsuspension separat abgeführt werden und bei dem kontinuierlich bestimmte Zellen der Zellsuspension konzentriert werden, dadurch gekennzeichnet, daß die konzentrierten Zellen in einer Waschlösung resuspendiert werden und daß die restlichen Bestandteile von den Zellen abgetrennt werden.

2. Verfahren nach Anspruch 1, dadurch gekennzeichnet, daß die Zellsuspension Blut umfaßt.

3. Verfahren nach Anspruch 2, dadurch gekennzeichnet, daß die Zellen einem Patienten autotransfundiert werden.

4. Verfahren nach Anspruch 2 oder 3, dadurch gekennzeichnet, daß die konzentrierten Zellen aus Erythrozyten bestehen.

5. Verfahren nach Anspruch 4, dadurch gekennzeichnet, daß die Konzentrierung der Zellen auf Hämokritwerte von ca. 50 bis 70% erfolgt.

6. Verfahren nach Anspruch 4 oder 5, dadurch gekennzeichnet, daß der Restplasmagehalt auf < 5 % der anfänglichen Plasmamenge vermindert wird.

7. Verfahren nach einem der Ansprüche 2 bis 6, dadurch gekennzeichnet, daß als Waschlösung isotonische Kochsalzlösung oder Ringerlösung verwendet wird.

8. Separationsvorrichtung zur Durchführung eines Verfahrens zur Aufbereitung einer Zellsuspension, insbesondere zur Durchführung des Verfahrens nach einem der Ansprüche 1 bis 7, mit einer Zentrifuge, welche zumindest eine Separationskammer (1), eine Zulautleitung (3) Für die Zellsuspension. eine Ablaufleitung (4) für ein zu gewinnendes, hochreines Zellkonzentrat, zumindest eine Ablaufleitung (5, 7) für nicht benötigte Bestandteile und zumindest eine Zulautleitung (6) für eine Waschlösung aufweist, dadurch gekennzeichnet, daß die Separationskammer im wesentlichen in Form eines Ringkanals (2) mit im wesentlichen rechteckförmigen Querschnitt ausgebildet ist, wobei die radiale Ahmessung des Ringkanals (2) kleiner ist als der mittlere Durchmesser des Ringkanals (2), daß der Ringkanal (2) in Umtangsrichtung an zwei Enden geschlossen ist, wobei an einem Ende die Zulautleitung (3) für die Zellsuspension und am anderen Ende die Ablautleitung (4) für das zu gewinnende, hochreine Zellkonzentrat angeschlossen sind, und daß der Ringkanal (2) in Umfangsrichtung in drei Bereiche unterteilt ist, von denen ein erster Bereich (8) einer ersten Separation der Zellsuspension, ein zweiter Bereich (15, 17) einer Resuspendierung in einer Waschlösung und ein dritter Bereich (9) einer zweiten Separation der resuspendierten Zellen dient.

9. Separationsvorrichtung nach Anspruch 8, dadurch gekennzeichnet, daß die Zulaufleitung (6) für Waschlösung zur Durchleitung der Waschlösung im Gegenstromverfahren stromauf oder stromab der Ablaufleitung (7) für die verunreinigte Waschlösung angeordnet ist.

10. Separationsvorrichtung nach einem der Ansprüche 8 oder 9, dadurch gekennzeichnet, daß die Ablaufleitung (7) für die verunreinigte Waschlösung einstückig mit der Ablaufleitung (5) für die nicht benötigten Bestandteile ausgebildet ist.

11. Separationsvorrichtung nach einem der Ansprüche 8 bis 10, dadurch gekennzeichnet, daß die Separationskammer (1) radiärsymmetrisch oder spiralig oder doppelspiralig ausgebildet ist.

12. Separationsvorrichtung nach einem der Ansprüche 8 bis 11, dadurch gekennzeichnet, daß die Zulaufleitung (6) für die Waschlösung für eine Strömung in radialer Richtung von außen nach innen, entgegen dem Schwerefeld, angeordnet ist.

13. Separationsvorrichtung nach einem der Ansprüche 8 bis 12, dadurch gekennzeichnet, daß die Separationskammer (1) einteilig ausgebildet ist.

14. Separationsvorrichtung nach einem der Ansprüche 8 bis 12, dadurch gekennzeichnet, daß die Separationskammer (1) zweiteilig ausgebildet ist.

15. Separationsvorrichtung nach Anspruch 14, dadurch gekennzeichnet, daß die beiden Teile (8, 9) der zweiteiligen Separationskammer (1) durch eine Leitung (17) miteinander verbunden sind, und daß die Zulaufleitung (6) für die Waschlösung im Bereich dieser Leitung (17) mündet.

16. Separationsvorrichtung nach einem der Ansprüche 8 bis 15, dadurch gekennzeichnet, daß die Separationskammer (1) selbsttragend und starr ausgebildet ist.

17. Separationsvorrichtung nach einem der Ansprüche 8 bis 16, dadurch gekennzeichnet, daß die Separationskammer (1) als wiederverwertbares Einweg-Teil ausgebildet ist.

18. Separationsvorrichtung nach einem der Ansprüche 8 bis 17, dadurch gekennzeichnet, daß die Zulaufleitung (6) für die Waschlösung im Anschlußbereich an den Ringkanal (2) der Separationskammer (1) kegelförmig (19) sich erweiternd ausgebildet ist.

19. Separationsvorrichtung nach Anspruch 18, dadurch gekennzeichnet, daß der kegelförmig sich erweiternde Bereich (19) der Zulaufleitung (6) für die Waschlösung über die gesamte Höhe des Ringkanals (2) mittels eines Hohlstegs (20) mit diesem verbunden ist.

20. Separationsvorrichtung nach Anspruch 18, dadurch gekennzeichnet, daß im Bereich der Mündung des den kegelförmigen Bereich (19) der Zulaufleitung (6) mit dem Ringkanal (2) verbindenden Hohlstegs (20) in Richtung des Separationsverlaufs hinter der Mündung eine in den Innenraum des Ringkanals (2) gerichtete Einwölbung (21) angeordnet ist.

## Claims

1. Process for the continuous preparation of a cell suspension in which the cell suspension is centrifuged and the separation constituents of the cells suspension are conducted away separately and in which continuously determined cells of the cell suspension are concentrated, characterised in that the concentrated cells are resuspended in a washing solution and that the residual constituents are separated from the cells.

2. Process according to claim 1, characterised in that the cell suspension includes blood.

3. Process according to claim 2, characterised in that the cells are auto transferred to a patient.

4. Process according to claim 2 or 3, characterised in that the concentrated cells consist of erythrocytes.

5. Process according to claim 4, characterised in that the concentration of the cells takes place at hemocrit values of about 50 to 70%.

6. Process according to claim 4 or 5, characterised in that the residual plasma content is reduced to <5% of the initial quantity of plasma.

7. Process according to one of the claims 2 to 4, characterised in that as washing solution isotonic sodium chloride solution or Ringer solution is used.

8. Separation apparatus for carrying out a process for the preparation of a cell suspension in particular for carrying out the process according to one of the claims 1 to 7, with a centrifuge which has at least one separation chamber (1), a feed pipe (3) for the cell suspension, a flow off pipe (4) for a highly pure cell concentrate to be obtained, at least one flow off pipe (5,7) for constituents not required and at least one feed pipe (6) for a washing solution, characterised in that the separating chamber is formed essentially in the shape of an annular channel (2) with essentially rectangular cross section whereby the radial dimension of the annular channel (2) in a washing solution is smaller than the middle diameter of the annular channel (2), that the annular channel (2) in peripheral direction is closed at two ends whereby at one end the feed pipe (3) for the cell suspension and at the other end the flow off pipe (4) for the highly pure cell concentrate to be obtained are connected and that the annular channel (2) in peripheral direction is divided into three areas of which a first area (8) serves a first separation of the cell suspension, a second area (15,17) a resuspension in a washing solution and a third area (9) a second separation of the resuspended cells.

9. Separation apparatus according to claim 8, characterised in that the feed pipe (6) for washing solution is arranged for conducting through the washing solution in the counter flow process upstream or downstream of the flows off pipe (7) for the soiled washing solution.

10. Separation apparatus according to one of the claims 8 or 9, characterised in that flow off pipe (7) for the soiled washing solution is formed in one piece with the flow off pipe (5) for the constituents not required.

11. Separation apparatus according to one of the claims 8 to 10, characterised in that the separation chamber (1) is formed radial symmetric or spirally or double spirally.

12. Separation apparatus according to one of the claims 8 to 11, characterised in that the feed pipe (6) for the washing solution is disposed in radial direction from outside inwards against the filed of gravity.

13. Separation apparatus according to one of the claims 8 to 12, characterised in that the separation chamber (1) is formed as one part.

14. Separation apparatus according to one of the claims 8 to 12, characterised in that the separation chamber (1) is formed in two parts.

15. Separation apparatus according to claim 14, characterised in that the two parts (8,9) of the two part separation chamber (1) are connected to one another by a pipe (17) and that the feed pipe (6) for the washing solution leads in the area of this conduit (17).

16. Separation apparatus according to one of the claims 8 to 15, characterised in that the separation chamber (1) is formed self supporting and rigid.

17. Separation apparatus according to one of the claims 8 to 16, characterised in that the separation chamber (1) is formed as a reusable one way part.

18. Separation apparatus according to one of the claims 8 to 17, characterised in that the feed pipe (6) for the washing solution is formed in the connection area on the annular channel (2) of the separation chamber (1), widening conically (19).

19. Separation apparatus according to claim 18, characterised in that the conically widening area (19) of the feed conduit (6) for the washing solution is connected to this over the whole height of the annular ring (2) by means of a hollow cross piece (20).

20. Separation apparatus according to claim 18, characterised in that in the area of the mouth of the hollow cross piece (20) connecting the conical area (19) of the feed pipe (6) to the annular channel (2) there is arranged in direction of the separation course behind the mouth a curvature (21) directed into the inner space of the annular channel (2).

## Revendications

1. Procédé de traitement en continu d'une suspension cellulaire, selon lequel on centrifuge la suspension cellulaire et on évacue séparément les constituants séparés de la suspension cellulaire, et selon lequel on concentre en continu certaines cellules de la suspension cellulaire, caractérisé en ce que l'on remet les cellules concentrées en suspension dans une solution de lavage et en ce que l'on sépare les constituants résiduels des cellules.

2. Procédé selon la revendication 1, caractérisé en ce que la suspension cellulaire comprend du sang.

3. Procédé selon la revendication 2, caractérisé en ce que les cellules sont autotransfusées à un malade.

4. Procédé selon la revendication 2 ou 3, caractérisé en ce que les cellules concentrées sont constituées d'érythrocytes.

5. Procédé selon la revendication 4, caractérisé en ce que la concentration des cellules s'effectue à des valeurs de l'hématocrite d'environ 50 à 70 %.

6. Procédé selon la revendication 4 ou 5, caractérisé en ce que la teneur en plasma résiduel est réduite à moins de 5 % de la quantité de plasma de départ.

7. Procédé selon l'une des revendications 2 à 6, caractérisé en ce que l'on utilise comme solution de lavage une solution isotonique de chlorure de sodium ou une solution de Ringer.

8. Dispositif de séparation pour la mise en oeuvre d'un procédé de traitement d'une suspension cellulaire, en particulier pour la mise en oeuvre du procédé selon l'une des revendications 1 à 7, comportant une centrifugeuse qui présente au moins une chambre de séparation (1), une conduite d'amenée (3) pour la suspension de cellules, une conduite de sortie (4) pour le concentré de cellules très pur à obtenir, au moins une conduite de sortie (5, 7) pour les constituants non nécessaires et au moins une conduite d'amenée (6) pour une solution de lavage, caractérisé en ce que la chambre de séparation est essentiellement formée d'un canal annulaire (2) de section essentiellement rectangulaire, la dimension radiale du canal annulaire (2) étant inférieure au diamètre moyen du canal annulaire (2), en ce que le canal annulaire (2) est fermé aux deux extrémités dans le sens du périmètre, la conduite d'amenée (3) de la suspension cellulaire étant raccordée à une extrémité et la conduite de sortie (4) pour le concentré cellulaire très pur à obtenir étant raccordée à l'autre extrémité, et en ce que le canal annulaire (2) est subdivisé dans le sens du périmètre en trois zones, la première zone (8) servant à une première séparation de la suspension cellulaire, la deuxième zone (14, 17) à la remise en suspension dans une solution de lavage et la troisième zone (9) à une seconde séparation des cellules remises en suspension.

9. Dispositif de séparation selon la revendication 8, caractérisé en ce que la conduite d'amenée (6) pour la solution de lavage est disposée en amont ou en aval de la conduite de sortie (7) de la solution de lavage contaminée pour faire passer la solution de lavage à contre-courant.

10. Dispositif de séparation selon l'une des revendications 8 ou 9, caractérisé en ce que la conduite de sortie (7) pour la solution de lavage contaminée forme une seule pièce avec la conduite de sortie (5) pour les constituants non nécessaires.

11. Dispositif de séparation selon l'une des revendications 8 à 10, caractérisé en ce que la chambre de séparation (1) a une configuration radialement symétrique ou en spirale ou en double spirale.

12. Dispositif de séparation selon l'une des revendications 8 à 11, caractérisé en ce que la conduite d'amenée (6) de la solution de lavage est disposée de façon à produire un courant dans le sens radial de l'extérieur vers l'intérieur, opposé au champ de pesanteur.

13. Dispositif de séparation selon l'une des revendications 8 à 12, caractérisé en ce que la chambre de séparation (1) est en une partie.

14. Dispositif de séparation selon l'une des revendications 8 à 12, caractérisé en ce que la chambre de séparation (1) est en deux parties.

15. Dispositif de séparation selon la revendication 14, caractérisé en ce que les deux parties (8, 9) de la chambre de séparation en deux parties (1) sont reliées entre elles par une conduite (17) et en ce que la conduite d'amenée (6) pour la solution de lavage débouche dans la zone de cette conduite (17).

16. Dispositif de séparation selon l'une des revendications 8 à 15, caractérisé en ce que la chambre de séparation (1) a une structure rigide et autoporteuse.

17. Dispositif de séparation selon l'une des revendications 8 à 16, caractérisé en ce que la chambre de séparation (1) est constituée d'un élément jetable et recyclable.

18. Dispositif de séparation selon l'une des revendications 8 à 17, caractérisé en ce que la conduite d'amenée (6) pour la solution de lavage s'élargit en cône (19) dans la zone de raccordement au canal annulaire (2) de la chambre de séparation (1).

19. Dispositif de séparation selon la revendication 18, caractérisé en ce que la zone s'élargissant en cône (19) de la conduite d'amenée (6) pour la solution de lavage est reliée au canal annulaire (2) sur toute la hauteur par une barrette creuse (20).

20. Dispositif de séparation selon la revendication 18, caractérisé en ce que, dans la zone de l'ouverture de la barrette creuse (20) reliant la zone conique (19) de la conduite d'amenée (6) au canal annulaire (2), est disposée une moulure (21) orientée dans le sens du déroulement de la séparation dans la chambre interne du canal annulaire (2) derrière l'ouverture.
